(19) Europäisches Patentamt
European Patent Office
Office européen des brevets

(11) Veröffentlichungsnummer: **0 579 638 B1**

(12) # EUROPÄISCHE PATENTSCHRIFT

(45) Veröffentlichungstag der Patentschrift: **26.10.94**

(51) Int. Cl.⁵: **C09B 23/10**, A61K 49/00, G11B 7/24, G03G 5/09

(21) Anmeldenummer: **92907490.4**

(22) Anmeldetag: **28.03.92**

(86) Internationale Anmeldenummer:
**PCT/EP92/00693**

(87) Internationale Veröffentlichungsnummer:
**WO 92/17549 (15.10.92 92/26)**

(54) **POLYKETOMETHINFARBSTOFFE.**

(30) Priorität: **06.04.91 DE 4111159**

(43) Veröffentlichungstag der Anmeldung:
**26.01.94 Patentblatt 94/04**

(45) Bekanntmachung des Hinweises auf die Patenterteilung:
**26.10.94 Patentblatt 94/43**

(84) Benannte Vertragsstaaten:
**CH DE FR GB IT LI NL**

(56) Entgegenhaltungen:
**WO-A-89/12080**
**WO-A-40/0101**
**BE-A- 660 252**
**FR-A- 1 401 589**
**US-A- 5 002 812**

**Japanese Patents Abstracts, Section CH, Week 8945, 1989, Derwent Publications Ltd, (London, GB) Class G, page 2, No. 89-327504/45**

(73) Patentinhaber: **BASF Aktiengesellschaft**
**Carl-Bosch-Strasse 38**
**D-67063 Ludwigshafen (DE)**

(72) Erfinder: **ALBERT, Bernhard**
**Rietburgstrasse 13**
**D-6701 Maxdorf (DE)**
Erfinder: **KESSEL, Knut**
**Berliner Strasse 18**
**D-6800 Mannheim 1 (DE)**
Erfinder: **MARTIN, Hans-Dieter**
**Haafstrasse 13**
**D-8700 Würzburg (DE)**
Erfinder: **SILBER, Stefan**
**Hermann-Schumacher-Strasse 20**
**D-4150 Krefeld 1 (DE)**

EP 0 579 638 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Vorliegende Erfindung betrifft neue Polyketonmethinfarbstoffe der allgemeinen Formel I

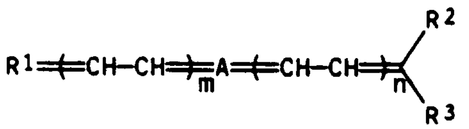

I

in der die Variablen folgende Bedeutung haben:

A eine Gruppierung der Formel A1 oder A2

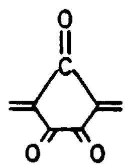

A1

oder

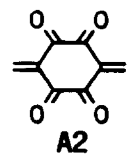

A2

$R^1$ einen 5- oder 6-gliedrigen cycloaliphatischen Rest mit ein oder zwei Heteroatomen aus der Gruppe $-NR^4-$, -O- und -S-, der mit einer iso- oder heteroaromatischen Gruppe anelliert sein kann, wobei

$R^4$ folgende Bedeutung hat:
eine $C_5$-$C_7$-Cycloalkylgruppe oder eine Phenylgruppe, die Halogen, $C_1$-$C_4$-Alkyl oder -Alkoxy als Substituenten tragen kann;
eine $C_1$-$C_{20}$-Alkylgruppe, deren C-Kette durch ein bis fünf Sauerstoffatome in Etherfunktion unterbrochen sein kann und die folgende Substituenten tragen kann: Hydroxy, Phenyl, 1,3-Dioxan-2-yl, 1,3-Dioxolan-2-yl, $-NHR^5$, $-NHCOR^5$, $-CONHR^5$, $-OCONHR^5$, $-N^{\oplus}(R^5)_3 \bullet An^{\ominus}$, $-SO_3H$ oder $-SO_3^{\ominus}Ka^{\oplus}$,
wobei

$R^5$ einen der unsubstituierten oder durch Hydroxy oder Phenyl substituierten Alkylreste $R^4$ oder Phenyl bedeutet und die Reste $R^5$ im Fall des Substituenten $-N^{\oplus}(R^5)_3$ gleich oder verschieden sein können,

$An^{\ominus}$ für das Äquivalent eines Anions und

$Ka^{\oplus}$ für das Äquivalent eines Kations stehen;

$R^2$, $R^3$ gleiche oder verschiedene $C_1$-$C_{10}$-Alkylgruppen oder gemeinsam einen der Reste $R^1$, wobei im Fall der Gruppierung A1 Reste $R^1$ der Formeln

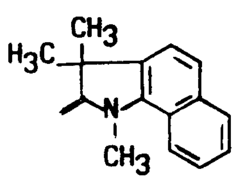

;

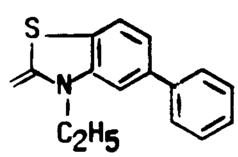

oder

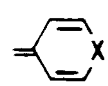

in denen X für Sauerstoff, Schwefel, Selen, $-N(CH_3)_2$ oder

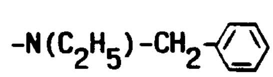

steht und die durch Alkyl-, Aryl- oder Aminogruppen substituiert sein können, ausgeschlossen sind, und
im Fall der Gruppierung A2 Reste $R^1$ der Formeln

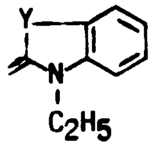 ; 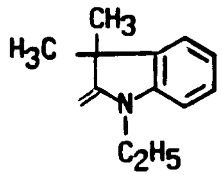 ; 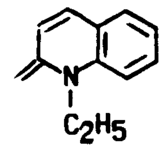 oder 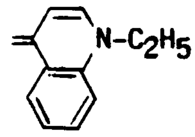

in denen Y für Sauerstoff oder Schwefel steht, ausgeschlossen sind;

m, n gleiche oder verschiedene ganzzahlige Werte von 0 bis 3.

Farbstoffe, die im Infrarotbereich absorbieren, sind für viele Anwendungszwecke von Interesse. Sie werden vielfach als Sensibilisatoren für elektrophotographische Schichten und für Photopolymerisationen sowie als gegen Laserlicht empfindliche Farbstoffe in optischen Aufzeichnungsmedien eingesetzt.

Eignen sich die Farbstoffe zusätzlich zur Erzeugung von Singulett-Sauerstoff, so ergeben sich weitere interessante Einsatzmöglichkeiten in der photodynamischen Tumortherapie, die auf der selektiven Anreicherung von Farbstoffen in Krebsgewebe basiert und sowohl eine genaue Lokalisierung als auch eine wirkungsvolle Behandlung schon im Frühstadium des Tumors ermöglicht. Die Bestrahlung von mit Farbstoff angereichertem Tumorgewebe führt, wahrscheinlich über die Bildung von Singulett-Sauerstoff, zu dessen selektiver Zerstörung. Da die Lichtdurchlässigkeit von Körpergewebe mit wachsender Wellenlänge stark ansteigt und für IR-Strahlung von 800 nm um einige Zehnerpotenzen größer ist als für sichtbares Licht, sind als Strahlungsquellen Halbleiterlaser, die Licht im nahen IR-Bereich emittieren, und Farbstoffe, die in diesem Bereich hohe Absorption aufweisen, für die Tumortherapie besonders geeignet.

In der US-A-5 002 812 und der JP-A-242 288 (1989) ist die Verwendung von Methinfarbstoffen mit Cyclopentantriongruppierung auf der Basis von benzanelliertem Indolin bzw. von 3-Ethylbenzthiazol und Pyridin-Pyran- und Thiopyranen in optischen Aufzeichnungsmedien beschrieben.

Aus der US-A-3 140 951 sind Methinfarbstoffe mit Cyclohexantetrongruppierung auf der Basis von 3-Ethyl-benzoxazol und -benzthiazol, 1-Ethyl-3,3-dimethyl-2-indol und 1-Ethyl-2(1H)- und -4(1H)chinolin bekannt, die hier jedoch als Desensibilisatoren für photographische Emulsionen eingesetzt werden.

Der Erfindung lag die Aufgabe zugrunde, Farbstoffe zu finden, die im Infrarotbereich absorbieren und günstige Anwendungseigenschaften besitzen.

Demgemäß wurden die eingangs definierten Polyketomethinfarbstoffe I gefunden.

Der Rest $R^1$ bedeutet dabei 5- oder 6-gliedrige cycloaliphatische Reste mit ein oder zwei Heteroatomen aus der Gruppe $-NR^4-$, -O-und -S-, die mit einer iso- oder heteroaromatischen Gruppe linear oder angular anelliert sein können. Als Beispiele für bevorzugte Reste $R^1$ seien genannt:

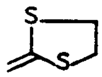
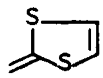
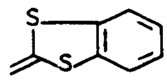

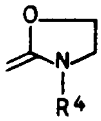
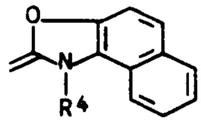
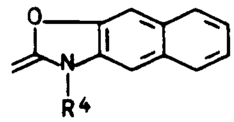

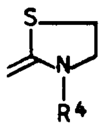
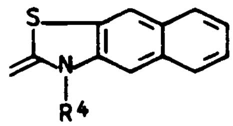

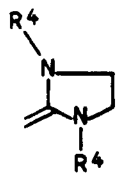
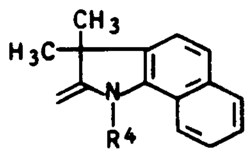

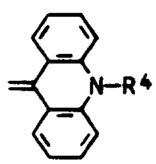
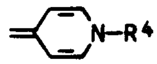
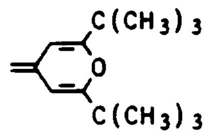

Beispiele für besonders bevorzugte Reste $R^1$ sind:

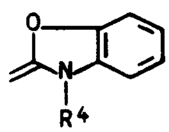
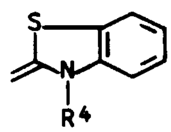
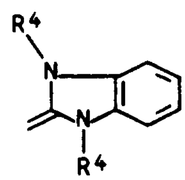

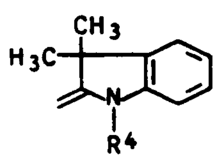
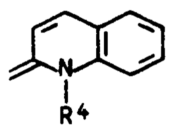
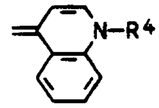

Ganz besonders bevorzugter Rest $R^1$ ist

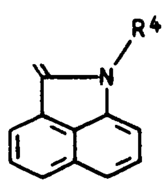

Die anellierten Benzol- und Naphthalinkerne sind dabei vorzugsweise unsubstituiert. Sie können aber auch $C_1$-$C_5$-Alkyl, -Alkoxy, Halogen, $-SO_3H$ oder $-SO_3^{\ominus}Ka^{\oplus}$ als Substituenten tragen.

Als Rest $R^4$ eignen sich $C_1$-$C_{20}$-Alkylgruppen wie Methyl, Ethyl, Propyl, Isopropyl, Butyl, Isobutyl, sec.-Butyl, tert.-Butyl, Pentyl, Isopentyl, Neopentyl, tert.-Pentyl, Hexyl, 2-Methylpentyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Nonadecyl und Eicosyl sowie verzweigte Reste dieser Art. Dabei sind die $C_1$-$C_{12}$-Alkylgruppen bevorzugt und die $C_1$-$C_6$-Alkylgruppen besonders bevorzugt.

Die C-Kette dieser Alkylgruppen kann durch Sauerstoffatome in Etherfunktion unterbrochen sein. Dabei enthalten $C_1$-$C_6$-Alkylgruppen in der Regel ein oder zwei, $C_7$-$C_{12}$-Alkylgruppen bis zu drei und $C_{13}$-$C_{20}$-Alkylgruppen bis zu fünf Sauerstoffatome in der Kette. Als Beispiele seien die folgenden Gruppierungen genannt: bevorzugt 2-Methoxy-, 2-Ethoxy-, 2-Propoxy-, 2-Isopropoxy- und 2-Butoxyethyl, daneben 2- und 3-Methoxypropyl, 2- und 3-Ethoxypropyl, 2- und 4-Ethoxy-butyl, 2- und 4-Isopropoxybutyl, 5-Ethoxypentyl, 6-Methoxyhexyl, 4-Oxa-6-ethyldecyl, bevorzugt 3,6-Dioxaheptyl, 3,6-Dioxaoctyl, 3,6-Dioxadecyl, 3,6,9-Trioxadecyl und 3,6,9-Trioxaundecyl.

Die Alkylreste $R^4$ können außerdem Substituenten wie Hydroxy, Phenyl, 1,3-Dioxan-2-yl und 1,3-Dioxolan-2-yl tragen. Beispiele sind (dabei bedeutet Ph = Phenyl):

$-(CH_2)_2$-OH, $-(CH_2)_3$-OH, $-CH_2$-CH(OH)-$CH_3$, $-(CH_2)_4$-OH,
$-CH_2$-CH(OH)-$CH_2CH_3$,
$-(CH_2)_5$-OH, $-(CH_2)_6$-OH, $-(CH_2)_7$-OH, $-(CH_2)_8$-OH und $-(CH_2)_{11}$-OH;
$-CH_2$-Ph, $-(CH_2)_2$-Ph, -CH(Ph)-$CH_3$ und -[$(CH_2)_2$-O]$_2$-$CH_2$-Ph;

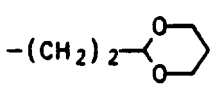, 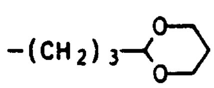, 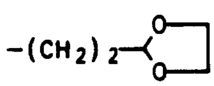 und 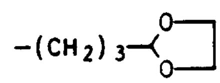.

Weitere mögliche Substituenten für die Alkylreste $R^4$ sind solche der Formeln -NH-$R^5$, -NH-CO-$R^5$, -CO-NH-$R^5$ und -O-CO-NH-$R^5$, die bevorzugt in $\omega$-Stellung stehen. $R^5$ bedeutet dabei einen der unsubstituierten oder durch Hydroxy oder Phenyl substituierten Alkylreste $R^4$, dessen C-Kette durch Sauerstoffatome in Etherfunktion unterbrochen sein kann, oder Phenyl. Beispiele für den Rest $R^5$ sind die entsprechenden oben aufgeführten Gruppen. Derart substituierte Reste $R^4$ sind beispielsweise:

$-(CH_2)_2$-NH-$CH_3$, $-(CH_2)_3$-NH-$C_2H_5$, -CH($CH_3$)-CH($CH_3$)-NH-$C_2H_5$,
$-(CH_2)_4$-NH-$C_3H_7$, $-(CH_2)_3$-NH-CH$(CH_3)_2$, $-(CH_2)_2$-NH-$C_4H_9$ und
$-(CH_2)_2$-NH-Ph;
-CH($CH_3$)-CH($CH_3$)-NH-CO-$CH_3$, $-(CH_2)_4$-NH-CO-$C_2H_5$,
$-(CH_2)$3-NH-CO-$C_3H_7$,
$-(CH_2)_2$-NH-CO-CH$(CH_3)_2$, $-(CH_2)_2$-NH-CO-$C_4H_9$, $-(CH_2)_2$-CO-NH-C$(CH_3)_3$ und
$-(CH_2)_2$-NH-CO-Ph;
-CH($CH_3$)-CH($CH_3$)-CO-NH-$CH_3$, $-(CH_2)_4$-CO-NH-$C_2H_5$,
$-(CH_2)_3$-CO-NH-$C_3H_7$,
$-(CH_2)_2$-CO-NH-CH$(CH_3)_2$, $-(CH_2)_2$-CO-NH-$C_4H_9$, $-(CH_2)_2$-CO-NH-C$(CH_3)_3$ und
$-(CH_2)_2$-CO-NH-Ph;
$-(CH_2)_4$-O-CO-NH-$CH_3$, $-(CH_2)_3$-O-CO-NH-$C_2H_5$, $-(CH_2)_3$-O-CO-NH-$C_3H_7$,
$-(CH_2)_3$-O-CO-NH-CH$(CH_3)_2$, $-(CH_2)_3$-O-CO-NH-$C_4H_9$,
$-(CH_2)_3$-O-CO-NH-C$(CH_3)_3$
und $-(CH_2)_2$-O-CO-NH-Ph.

Substituenten für die Alkylreste $R^4$ können schließlich auch solche der Formeln $-N^{\oplus}(R^5)_3 \bullet An^{\ominus}$, $-SO_3H$ oder $-SO_3^{\ominus} \bullet Ka^{\oplus}$ sein, die ebenfalls bevorzugt in $\omega$-Stellung stehen. Die Reste $R^5$ können dabei gleich oder verschieden sein. Beispiele für diese Reste $R^4$ sind:

$-(CH_2)_2$-$N^{\oplus}(C_2H_5)_3$, $-(CH_2)_3$-$N^{\oplus}(CH_3)_3$, $-(CH_2)_3$-$N^{\oplus}(C_2H_5)_3$,
$-(CH_2)_3$-$N^{\oplus}(C_4H_9)_3$,
$-(CH_2)_4$-$N^{\oplus}(C_2H_5)_3$, $-(CH_2)_6$-$N^{\oplus}(C_2H_5)_3$, $-(CH_2)_{10}$-$N^{\oplus}(C_2H_5)_3$,
$-(CH_2)_2$-$N^{\oplus}(CH_3)(C_2H_5)_2$ und $-(CH_2)_2N^{\oplus}(CH_3)(C_2H_5)(C_3H_7)$;
$-(CH_2)_2$-$SO_3H$, $-(CH_2)_3$-$SO_3H$, $-(CH_2)_4$-$SO_3H$, $-(CH_2)_6$-$SO_3H$ und
$-(CH_2)_{10}$-$SO_3H$;
$-(CH_2)_2$-$SO_3^{\ominus}$, $-(CH_2)_3$-$SO_3^{\ominus}$, $-(CH_2)_4$-$SO_3^{\ominus}$, $-(CH_2)_6$-$SO_3^{\ominus}$ und
$-(CH_2)_{10}$-$SO_3^{\ominus}$.

Geeignete Anionen $An^{\ominus}$ können dabei Anionen anorganischer oder organischer Säuren sein. Besonders bevorzugt sind z.B. Chlorid, Bromid und Sulfat sowie Maleat, Fumarat, Tosylat und Salicylat.

Geeignete Kationen $Ka^{\oplus}$ können dabei Alkalimetallionen wie besonders $Na^{\oplus}$ und $K^{\oplus}$ sein. Besonders bevorzugt sind Trialkylammoniumionen der Formel $HN^{\oplus}(R^5)_3$, beispielsweise solche mit den oben aufgeführten Gruppen $-N^{\oplus}(R^5)_3$.

Daneben sind als Rest $R^4$ geeignet Cyclopentyl, Cyclohexyl, Cycloheptyl und Phenyl, das Halogen, $C_1$-$C_4$-Alkyl oder -Alkoxy als Substituenten tragen kann, wie: 2-, 3- und 4-Chlorphenyl, 2,4- und 2,6-Dichlorphenyl, 2-, 3- und 4-Bromphenyl, 2-, 3- und 4-Methylphenyl, 2,4-Dimethylphenyl, 2-, 3- und 4-Methoxyphenyl und 2,4-Dimethoxyphenyl.

Als Reste $R^2$ oder $R^3$ sind die für den Rest $R^4$ aufgeführten Alkylgruppen mit bis zu 10 C-Atomen geeignet, wobei $R^2$ und $R^3$ vorzugsweise gleiche Alkylgruppen bedeuten und Methyl besonders bevorzugt ist.

Die Reste $R^2$ und $R^3$ können aber auch gemeinsam einen der oben genannten Reste $R^1$ bedeuten. Dabei sind sowohl unsymmetrisch als auch symmetrisch aufgebaute Methinfarbstoffe möglich, wobei die letzteren bevorzugt sind.

Die variablen m und n sind gleich oder verschieden und können die Werte 0, 1, 2 oder 3 annehmen. m bedeutet dabei bevorzugt 0 und besonders bevorzugt 1. Handelt es sich bei den Resten $R^2$ und $R^3$ um Alkylgruppen, so steht n vorzugsweise für 0. Bilden die Reste $R^2$ und $R^3$ gemeinsam einen Rest $R^1$, so nimmt n bevorzugt den gleichen Wert wie m an.

Methoden zur Herstellung von Methinfarbstoffen mit Cyclohexantetrongruppierung sind allgemein bekannt und beispielsweise in der US-A-3 140 951 beschrieben.

Die erfindungsgemäßen Polyketomethinfarbstoffe I können analog dargestellt werden, beispielsweise durch Kondensation der die Gruppen A repräsentierenden Verbindungen 1,2,4-Cyclopentantrion oder 2,5-Dihydroxy-p-benzochinon mit den die Gruppierungen

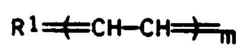

repräsentierenden, mit einer Abgangsgruppe X versehenen heterocyclischen Ammoniumsalzen der Formel IIa

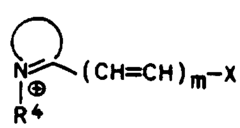

IIa

die beispielsweise in Houben-Weyl, 4. Aufl., Band V/1d, S. 239 bis 240 (1972) oder in J. Heterocyclic Chem. 22, S. 1727 bis 1734 (1985) beschrieben sind.

Für den Fall m = 0 bedeutet X vorzugsweise eine Methylmercaptogruppe, für die Fälle $m \geqq 1$ ist die Acetanilidogruppe als Abgangsgruppe bevorzugt.

Eine weitere Möglichkeit zur Einführung der Gruppierung

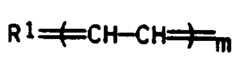

besteht darin, die entsprechenden Aldehyde der Formel IIb

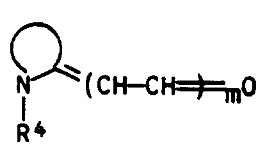

IIb

bei der Kondensation einzusetzen.

Die Verbindungen IIa und IIb sind durch Umsetzung der jeweiligen am Stickstoff unsubstituierten Heterocyclen mit einer Verbindung $R^4$-Y erhältlich, wobei Y beispielsweise für Chlor, Brom, Iod, $-O-SO_2-Ph-4-CH_3$, $-O-SO_2-CH_3$ oder $-O-SO_2-CF_3$ steht.

Für den Fall m = 0 können auch die aktivierten dimeren Heterocyclen, beispielsweise solche der Formeln

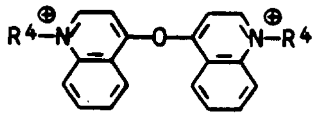

oder

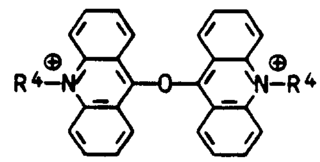

eingesetzt werden.

Umgekehrt kann die Kondensation, analog zur US-A-3 140 951 oder zu Houben-Weyl, 4. Aufl., Band V/1d, S. 239 bis 290 (1972), auch mit 1,2,4-Cyclopentantrion oder 1,2,4,5-Cyclohexantetron, das jeweils zweifach durch Anilinomethylen (Abgangsgruppe -NH-Ph) substituiert ist, und den in 2-Stellung methylierten heterocyclischen Ammoniumsalzen erfolgen (m = 1).

Zum Aufbau der Polymethinkette (m = 2 oder 3) können die in 2-Stellung methylierten heterocyclischen Salze wie z.B. in Houben-Weyl, 4. Aufl., Band V/1d, S. 268 bis 274 (1972) beschrieben, beispielsweise mit Verbindungen der Formel

$$Ph{-}N{-}CH{=}\!(\!{=}CH{-}CH{=}\!)_{m'}\,NH{-}Ph\cdot HCl$$

oder HBr (m' = 1 oder 2) in Acetanhydrld zu einem Hemicyanin der Formel

$$R^1{=}\!(\!{=}CH{-}CH{=}\!)_{m''}N(Ph){-}COCH_3$$

(m" = 2 oder 3) umgesetzt werden, das dann zur weiteren Kondensation mit der Gruppe A eingesetzt werden kann.

Zur Herstellung der ebenfalls erfindungsgemäßen unsymmetrischen Polyketomethinfarbstoffe der Formel I, in der $R^2$ und $R^3$ Alkylreste bedeuten, werden in 5- bzw. 6-Stellung zweifach alkylierte 1,2,4-Cyclopentantrione bzw. 2,5-Dihydroxy-p-benzochinone als Ausgangsprodukte für die Kondensation verwendet.

Die Kondensation wird zweckmäßigerweise in einem Lösungsmittel, wie Diethylether, Tetrahydrofuran, Trichlorethylen, 1,1,1-Trichlorethan, Methylenchlorid, Chloroform, Methanol, Ethanol, Propanol, Isopropanol, Butanol, Eisessig, Propionsäure und Acetonitril, vorzugsweise unter Zusatz eines Katalysators, wie Ammoniumacetat, Piperidin und Pyridin oder deren Acetaten bei Temperaturen zwischen 20 °C und der Siedetemperatur des verwendeten Lösungsmittels durchgeführt.

Bevorzugt ist die Verwendung von tertiären Aminen wie Tripropylamin, Tributylamln, besonders Triethylamln, Picolinen, Lutidinen, besonders Pyridin, oder deren Mischungen als Lösungsmittel, da in diesen Fällen der Zusatz eines weiteren Katalysators unterbleiben kann.

Eine weitere Möglichkeit zur Herstellung von in 6-Stellung des Cyclohexantetrons dialkylierten Methinfarbstoffen I besteht in der Umsetzung des 5,5-Dialkyl-1,3-cyclohexandions mit einem heterocyclischen Ammoniumsalz mit Abgangsgruppe und anschließende selektive Oxidation des erhaltenen Kondensationsprodukts mit Selendioxid in einem Lösungsmittel wie Eisessig zur entsprechenden Tetraketoverbindung.

Die erfindungsgemäßen Polyketomethinfarbstoffe I weisen in einigen Fällen hohe Absorptionen im Infrarot-Bereich auf. Sie sind zur Erzeugung von Singulett-Sauerstoff gut geeignet und können daher beispielsweise in der photodynamischen Tumortherapie eingesetzt werden.

Außerdem eignen sie sich zur Verwendung als Sensibilisatoren in elektrophotographischen Aufzeichnungsmaterialien, wie sie beispielsweise in der EP-A-150 419 beschrieben sind. Geeignete einschichtige Systeme weisen dabei auf einem leitfähigen Trägermaterial vorzugsweise eine Schicht auf, die aus 45 bis 75 Gewichtsteilen eines Bindemittels, 30 bis 60 Gewichtsteilen einer Ladungsträger transportierenden Verbindung, gegebenenfalls bis zu 25 Gewichtsteilen eines weiteren, im wesentlichen inaktiven Bindemittels und 0,05 bis 0,8 Gewichtsteilen des Sensibilisators besteht, der bei aktinischer Belichtung Ladungsträger erzeugt. Diese Schicht wird vorteilhaft so aus einer etwa 6 gew.-%igen Lösung in einem geeigneten organischen Lösungsmittel auf das gereinigte leitfähige Trägermaterial aufgebracht, daß nach dem Ablüften des Lösungsmittels je nach Verwendungszweck eine Trockenschichtdicke von ca. 0,8 bis 40 µm, bei elektrophotographischen Druckformen insbesondere 0,8 bis 6 µm, resultiert. Weitere Einzelheiten zum Aufbau dieser Systeme sind der EP-A-150 419 zu entnehmen.

Weiterhin sind die Farbstoffe I als Sensibilisatoren für Photopolymerisationen, beispielsweise für die Lackhärtung geeignet. Nicht zuletzt können sie, wie für Azulenquadratsäurefarbstoffe in der US-A-4 904 566

beschrieben, als gegen Laserlicht empfindliche Farbstoffe in optischen Aufzeichnungsmedien eingesetzt werden.

Beispiele

a) Herstellung von Polyketomethinfarbstoffen I

a1) Herstellung von symmetrischen Triketomethinfarbstoffen Ia'

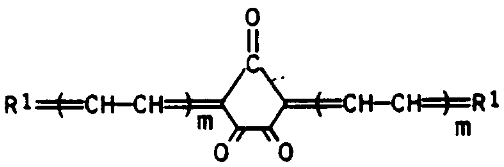

Ia'

Beispiel 1

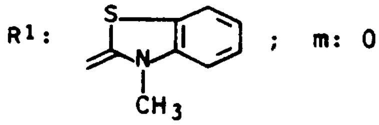

Eine Mischung aus 1,61 g (5 mmol) 3-Methyl-2-methylmercaptobenzothiazoliumiodid, 0,28 g (2,5 mmol) 1,2,4-Cyclopentantrion und 2 ml frisch destilliertem Triethylamin in 20 ml Ethanol wurde 30 min unter Rückfluß erhitzt.

Der beim Abschrecken entstandene Niederschlag wurde abgetrennt, nacheinander mit Methanol, Essigester und Ether gewaschen und anschließend zweimal aus Dimethylformamid umkristallisiert.

Es wurden 0,45 g 3,5-Di-(3-methyl-2-benzothiazolinyliden)-1,2,4-cyclopentantrion erhalten (Ausbeute 42 %):
Schmp.: > 250 °C; $\lambda_{max}$ = 467 nm ($CH_2Cl_2$).

Beispiel 2

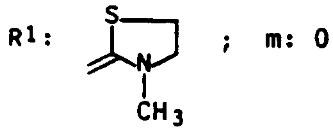

Die Umsetzung von 1,37 g (5 mmol) 3-Methyl-2-methylmercaptothiazoliniumiodid erfolgte analog zu Beispiel 1. Das Produkt wurde aus Acetonitril umkristallisiert.

Es wurden 0,35 g 3,5-Di-(3-methyl-2-thiazolinyliden)-1,2,4-cyclopentantrion erhalten (Ausbeute 45 %):
Schmp.: > 250 °C; $\lambda_{max}$ = 399 nm ($CH_2Cl_2$).

Beispiel 3

R1: 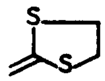 ; m: 0

Eine Mischung aus 1,31 g (5 mmol) 2-Methylmercapto-1,3-dithiolaniummethosulfat,0,28 g (2,5mmol) 1,2,4-Cyclopentantrion und 2 ml frisch destilliertem Triethylamin in 20 ml Pyridin wurde 45 min unter Rückfluß erhitzt.

Der beim Abkühlen entstandene Niederschlag wurde abgetrennt, nacheinander mit Methanol und Essigester gewaschen und aus Dimethylformamid umkristallisiert.

Es wurden 0,48 g 3,5-Di-(2-dithiolanyliden)-1,2,4-cyclopentantrion erhalten (Ausbeute 61 %): Schmp.: > 250 ° C; $\lambda_{max}$ = 446 nm (Dimethylformamid).

Beispiel 4 bis 7

Die in Tabelle 1 aufgeführten Triketomethinfarbstoffe Ia' wurden analog zu Beispiel 3 durch Umsetzung von 1,2,4-Cyclopentantrion mit 2-Methylmercapto-1,3-dithioliummethosulfat (Beispiel 4) bzw. mit dem entsprechenden in 2-Stellung durch einen Acetanilidovinylrest substituierten heterocyclischen Ammoniumiodid (Beispiel 5 bis 7) hergestellt.

Tabelle 1

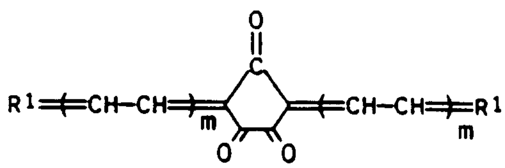 Ia'

| Bsp. | R1 | m | Ausbeute in g / % | Schmp. [°C] | $\lambda_{max}$ [nm] |
|---|---|---|---|---|---|
| 4 | 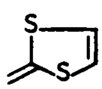 | 0 | 0,62 / 79* | >250 | 527 ($CH_2Cl_2$) |
| 5 | 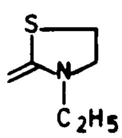 | 1 | 0,84 / 86 | >250 | 597 (Dimethylformamid) |
| 6 | 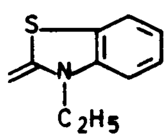 | 1 | 0,93 / 76 | >250 | 678 (Dimethylformamid) |
| 7 | 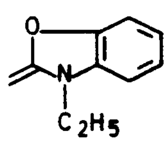 | 1 | 0,82 / 72 | >250 | 618 (Dimethylformamid) |

* Die Umkristallisation des Produktes erfolgte aus N,N-Dimethyl acetamid

Beispiel 8

$R^1$: 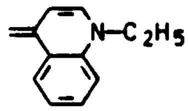 ; m: 1

Eine Mischung aus 1,52 g (5 mmol) 1-Ethyl-4-methylchinoliniumiodid, 0,80 g (2,5 mmol) 3,5-Dianilinomethylen-1,2,4-cyclopentantrion und 1 ml frisch destilliertem Triethylamin in 20 ml Acetonitril wurde 3 Tage unter Lichtausschluß bei Raumtemperatur gerührt.

Der entstandene Niederschlag wurde abgetrennt, mit Methanol gewaschen und aus N,N-Dimethylformamid umkristallisiert.

Es wurden 0,68 g 3,5-Di-((1-ethyl-4(1H)chinolinyliden)-ethyliden)-1,2,4-cyclopentantrion erhalten (Ausbeute 57 %):
Schmp.: > 250 °C; $\lambda_{max}$ = 816 nm (Dimethylsulfoxid).

Beispiel 9 bis 13

Die in Tabelle 2 aufgeführten Triketomethinfarbstoffe Ia'' wurden analog zu Beispiel 8 durch Umsetzung von 3,5-Dianilinomethylen-1,2,4-cyclopentantrion mit dem entsprechenden heterocyclischen Ammoniumiodid in ähnlichen Ausbeuten hergestellt.

Tabelle 2

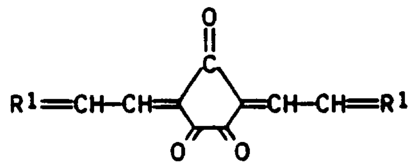

Ia"

| Bsp. | $R^1$ | $\lambda_{max}$ [nm] |
|---|---|---|
| 9 | 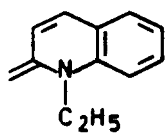 | 731 (Dimethylsulfoxid) |
| 10 | 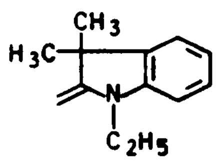 | 662 (Dimethylsulfoxid) |
| 11 | 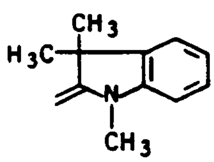 | 652 ($CH_2Cl_2$) |
| 12 | 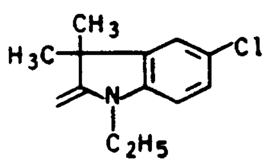 | 655 ($CH_2Cl_2$) |
| 13 | 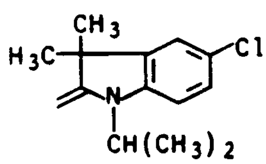 | 621 (Dimethylsulfoxid) |

Beispiel 14

$R^1$: 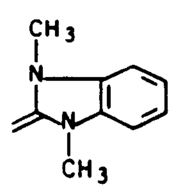 ; m: 0

Eine Mischung aus 1,46 g (5 mmol) 1,3-Dimethyl-2-methylmercaptobenzimidazoliumperchlorat, 0,28 g (2,5 mmol) 1,2,4-Cyclopentantrion und 2 ml frisch destilliertem Triethylamin in 20 ml Acetonitril wurde 2 h unter Rückfluß erhitzt.

Der beim Abkühlen entstandene Niederschlag wurde abgetrennt, mit Wasser gewaschen und aus N,N-Dimethylacetamid umkristallisiert.

Es wurden 0,23 g 3,5-Bis(1,3-dimethyl-2-benzimidazolinyliden)-1,2,4-cyclopentantrion erhalten (Ausbeute 23 %):
Schmp.: > 250 °C; $\lambda_{max}$ = 362 nm ($CH_2Cl_2$).

Beispiel 15

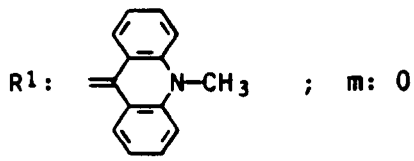

Eine Lösung aus 1,40 g (2 mmol) 9,9'-Oxy-bis(10-methylacridinium)-bis(trifluormethansulfonat) in 20 ml Acetonitril wurde bei Raumtemperatur zu einer Mischung aus 0,11 g (1 mmol) 1,2,4-Cyclopentantrion, 20 ml Acetonitril und 2 ml frisch destilliertem Triethylamin getropft.

Der entstandene Niederschlag wurde abgetrennt und mit viel Methylenchlorid gewaschen.

Es wurden 0,42 g 3,5-Bis(10-methyl-9-acridanyliden)-1,2,4-cyclopentantrion erhalten (Ausbeute 71 %):
Schmp.: > 250 °C; $\lambda_{max}$ = 788 nm ($CH_2Cl_2$).

a2) Herstellung von symmetrischen TetraketomethinfarbStoffen Ib'

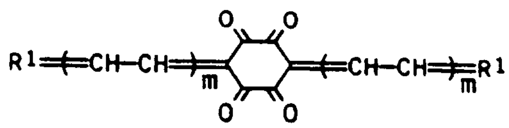 Ib'

Beispiel 16

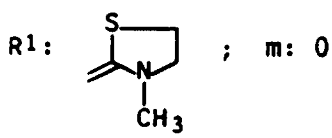

Eine Mischung aus 4 g (14,5 mmol) 3-Methyl-2-methylmercaptothiazoliniumiodld, 1 g (7,1 mol) 2,5-Dihydroxy-p-benzochinon und 2,5 ml frisch destilliertem Triethylamin in 40 ml Pyridin wurde 10 min unter Rückfluß erhitzt.

Der beim Abkühlen entstandene Niederschlag wurde abgetrennt und aus Dimethylformamid umkristallisiert.

Es wurden 1,09 g 3,6-Di-(3-methyl-2-thiazolinyliden)-1,2,4,5-cyclohexantetron erhalten (Ausbeute 45 %):
Schmp.: > 300 °C; $\lambda_{max}$ = 372 nm (Dimethylsulfoxid).

Beispiel 17

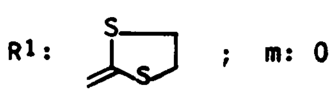

Eine Mischung aus 2,62 g (10 mmol) 2-Methylmercapto-1,3-dithiolaniummethosulfat, 0,70 g (5 mmol) 2,5-Dihydroxy-p-benzochinon und 2 ml frisch destilliertem Triethylamin in 30 ml Pyridin wurde 15 min unter Rückfluß erhitzt.

Der beim Abschrecken entstandene Niederschlag wurde abgetrennt, nacheinander mit Methanol und Aceton gewaschen und anschließend in heißem m-Kresol gelöst. Nach Filtration wurde die noch heiße

Lösung in einen Überschuß kaltes Methanol gegossen, wobei sich das Zielprodukt abschied.

Es wurden 1,02 g 3,6-Bis(1,3-dithiolan-2-yliden)-1,2,4,5-cyclohexantetron erhalten (Ausbeute 59 %): Schmp.:> 300 ° C; $\lambda_{max}$ = 415 nm (Dimethylsulfoxid).

Beispiel 18

R1: 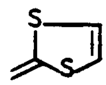 ; m: 0

Die Umsetzung von 2,60 g (10 mmol) 2-Methylmercapto-1,3-dithioliummethosulfat und die Isolierung des Produktes erfolgten analog zu Beispiel 17.

Es wurden 1,46 g 3,6-Bis(1,3-dithiol-2-yliden)-1,2,4,5-cyclohexantetron erhalten (Ausbeute 86 %):
Schmp.: > 300 ° C; $\lambda_{max}$ = 466 nm (Dimethylsulfoxid).

Beispiel 19

R1: 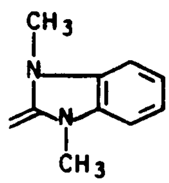 ; m: 1

Eine Mischung aus 2,88 g (10 mmol) 1,2,3-Trimethylbenzimidazoliumiodid und 0,69 g (2 mmol) 3,6-Dianilinomethylen-1,2,4,5-cyclohexantetron in 25 ml N,N-Dimethylformamid wurde 2 min unter Rückfluß erhitzt.

Der beim Abschrecken entstandene Niederschlag wurde abgetrennt, wiederholt mit Methanol gewaschen und aus N,N-Dimethylacetamid umkristallisiert.

Es wurden 0,43 g 3,6-Bis(2-(1,3-dimethyl-2-benzimidazolinyliden)ethyliden)-1,2,4,5-cyclohexantetron erhalten (Ausbeute 45 %):
Schmp.: > 280 ° C; $\lambda_{max}$ = 477 nm ($CH_2Cl_2$).

Beispiel 20

R1: 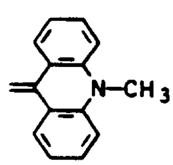 ; m: 0

Eine Lösung aus 1,40 g (5 mmol) 9,9'-Oxy-bis(10-methylacridinium)-bis(trifluormethansulfonat) in 20 ml Acetonitril wurde bei Raumtemperatur zu einer Mischung aus 0,14 g (1 mmol) 2,5-Dihydroxy-p-benzochinon, 20 ml Acetonitril und 2 ml frisch destilliertem Triethylamin getropft.

Der entstandene Niederschlag wurde abgetrennt und mit viel Methylenchlorid gewaschen.

Es wurden 0,38 g 3,6-Bis(10-methyl-9-acridanyliden)-1,2,4,5-cyclohexantetron erhalten (Ausbeute 61 %):
Schmp.: > 250 ° C; $\lambda_{max}$ = 693 nm ($CH_2Cl_2$).

Beispiel 21

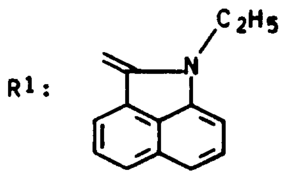

; m: 1

Eine Mischung aus 2,28 g (8,6 mmol) 2-Ethyl-1-methyl-benzo(c,d)-indoliumiodid, 1,00 g (2,9 mmol) 3,6-Dianilinomethylen-1,2,4,5-cyclohexantetron und 1 ml frisch destilliertem Triethylamin in 30 ml Pyridin wurde 10 min unter Rückfluß erhitzt.

Der beim Abschrecken entstandene Niederschlag wurde abgetrennt, nacheinander mit Methanol und Diethylether gewaschen und anschließend getrocknet.

Es wurden 1,4 g 3,6-Di-((2-ethyl-benzo(c,d)indolinyliden)-ethyliden)1,2,4,5-cyclohexantetron erhalten: $\lambda_{max}$ = 751 nm ($CH_2Cl_2$).

Beispiele 22 bis 28

Die in Tabelle 3 aufgeführten Tetraketomethinfarbstoffe Ib'' wurden analog zu Beispiel 21 durch Umsetzung von 3,6-Dianilinomethylen-1,2,4,5-cyclohexantetron mit dem entsprechenden heterocyclischen Ammoniumiodid hergestellt.

Tabelle 3

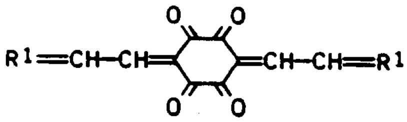

Ib"

| Bsp. | $R^1$ | $\lambda_{max}$ [nm] |
|---|---|---|
| 22 | 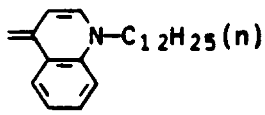 | 725 ($CH_2Cl_2$) |
| 23 | 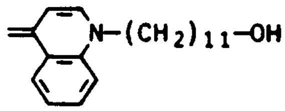 | 723 ($CH_2Cl_2$) |
| 24 | 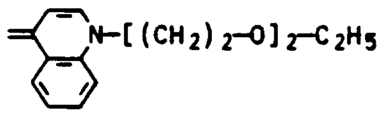 | 732 ($CH_2Cl_2$) |
| 25 | 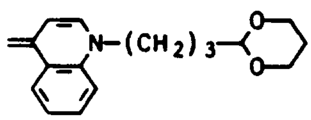 | 731 ($CH_2Cl_2$) |
| 26 | 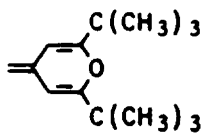 | 623 ($CH_2Cl_2$) |
| 27 | 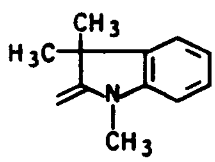 | 603 ($CH_2Cl_2$) |
| 28 | 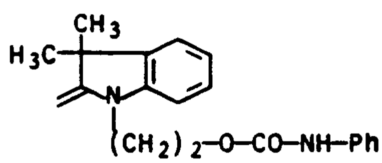 | 611 ($CH_2Cl_2$) |
| 29 | 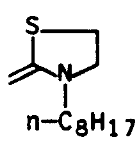 | 634 (Dimethylformamid) |

Beispiel 30

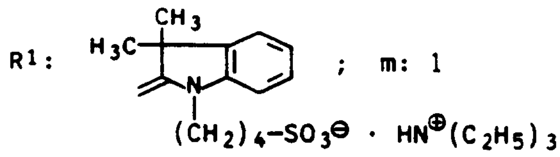

Eine Mischung aus 1,92 g (6,5 mmol) 2,3,3-Trimethylindolinium-1-(butan4-sulfonat), 1,00 g (2,9 mmol) Dianilinomethylen-1,2,4,5-cyclohexantetron und 2 ml frisch destilliertem Triethylamin in 30 ml Pyridin wurde 10 min unter Rückfluß erhitzt.

Der entstandene Niederschlag wurde nach Abkühlen abgetrennt, mit Essigester gewaschen und getrocknet.

Ausbeute: 0,25 g; Schmp.: 265 ° C.

a3) Herstellung von unsymmetrischen Tetraketomethinfarbstoffen Ic

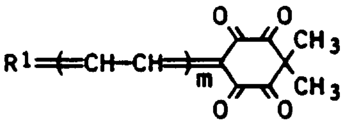

Ic

Beispiel 31

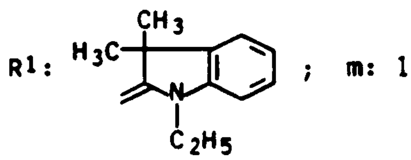

Eine Mischung aus 0,32 g (1 mmol) 1-Ethyl-2,3,3-trimethylindoliniumiodid, 0,27 g (1 mmol) 3-Anilinomethylen-6,6-dimethyl1,2,4,5-cyclohexantetron und 0,5 ml frisch destilliertem Triethylamin in 10 bis 15 ml Acetonitril wurde 3 Tage bei einer ölbadtemperatur von 50 ° C gerührt.

Der entstandene Niederschlag wurde abgetrennt, mit Wasser gewaschen und nach Trocknung aus Dioxan umkristallisiert.

Es wurden 0,19 g 6,6-Dimethyl-3-(2-(1-ethyl-3,3-dimethyl-2-indolinyliden)ethyliden)-1,2,4,5-cyclohexantetron erhalten (Ausbeute 52 %):

Schmp.: > 250 ° C; $\lambda_{max}$ = 507 nm ($CH_2Cl_2$).

Beispiele 32 bis 36

Die in Tabelle 4 aufgeführten Tetraketomethinfarbstoffe Ic wurden analog zu Beispiel 31 durch Umsetzung von 3-Anilinomethylen-6,6-dimethyl-1,2,4,5-cyclohexantetron mit dem entsprechenden heterocyclischen Ammoniumiodid hergestellt.

Tabelle 4

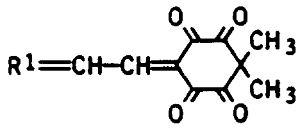

Ic

| Bsp. | $R^1$ | Ausbeute in g / % | Schmp. [°C] | $\lambda_{max}$ [nm] |
|---|---|---|---|---|
| 32 | 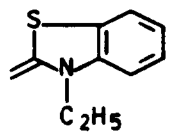 | 0,23 / 66 | >250 | 504 (Dimethylformamid) |
| 33 | 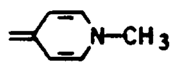 | 0,23 / 81 | >250 | 483 ($CH_2Cl_2$) |
| 34 | 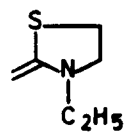 | 0,09 / 29* | >250 | 443 (Dimethylformamid) |
| 35 | 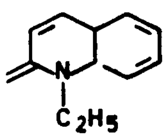 | 0,31 / 89** | >250 | 520 ($Cl_2Cl_2$) |
| 36 | 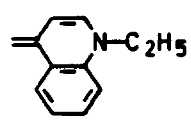 | 0,33 / 95** | >250 | 539 ($CH_2Cl_2$) |

* die Umkristallisation des Produktes erfolgte aus Ethanol

** die Umkristallisation des Produktes erfolgte aus N,N-Dimethylformamid

Beispiel 37

$R^1$: 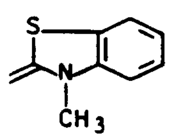 ; m: 0

Eine Mischung aus 8,62 g (0,03 mmol) 5,5-Dimethyl-2-(3-methylbenzothiazolinyliden)-1,3-cyclohexandion, das durch 1:1-Kondensation von 5,5-Dimethyl-1,3-cyclohexandion mit 3-Methyl-2-methylmercaptobenzothiazoliumiodid in siedendem 2-Propanol erhalten wurde, und 7,77 g (0,07 mol) Selendioxid in 50 ml Eisessig wurden 5 h bei einer ölbadtemperatur von 130 °C gerührt.

Das entstandene elementare Selen wurde abgetrennt, der Eisessig wurde am Rotationsverdampfer abgezogen. Der Rückstand wurde in Methylenchlorid aufgenommen und von unlöslichen Bestandteilen in der Siedehitze abfiltriert. Die verbleibende Lösung wurde auf -15 °C abgekühlt, wobei das Zielprodukt

auskristallisierte.

Es wurden 4,42 g 6,6-Dimethyl-3-(3-methylbenzothiazolinyliden)-1,2,4,5-cyclohexantetron erhalten (Ausbeute 47 %):

Schmp.: > 250 ° C; $\lambda_{max}$ = 389 nm (Dimethylsulfoxid).

b) Anwendungsbeispiele

b1) Erzeugung von Singulett-Sauerstoff

Die in den Tabellen 5 und 6 aufgeführten und analog zu den Beispielen a) hergestellten Farbstoffe I wurden, in dem jeweils angegebenen Lösungsmittel gelöst, mit einem Farbstofflaser durch Einstrahlen in die Absorptionsbande angeregt.

Die Bestimmung des gebildeten Singulett-Sauerstoffs erfolgte durch zeitaufgelöste Messung der Phosphorenz bei 1270 nm mit einer mit flüssigem Stickstoff gekühlten Germaniumdiode (Methode s. J. Am. Chem. Soc. 111, 2909-2914 (1989)); als Eichsubstanz diente 1,5-Diaminoanthrachinon.

**Tabelle 5**

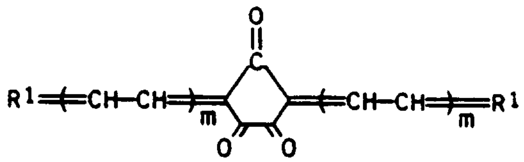

| Bsp. | R1 | m | Singulett-Sauerstoff Ausbeute [%] | λ [nm] |
|---|---|---|---|---|
| 1A | 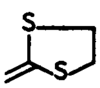 | 0 | 20,1 | 446 (Dimethyl-formamid) |
| 2A | 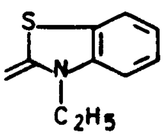 | 1 | 1,1 | 670 (Methanol) |

Tabelle 6

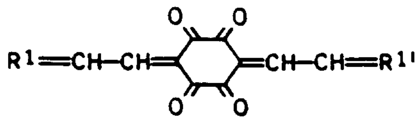

| Bsp. | $R^1$ | $R^{1'}$ | Singulett-Sauerstoff-Ausbeute [%] | λ [nm] |
|---|---|---|---|---|
| 3A | 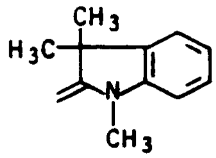 | $R^1$ | 8,3 | 590 ($CH_2Cl_2$) |
| 4A | 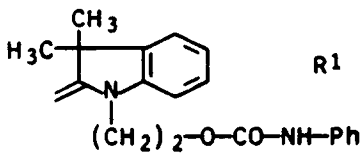 | $R^1$ | 4,7 | 590 (Ethanol) |
| 5A | 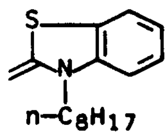 | $R^1$ | 4,4 | 634 (Dimethylformamid) |
| 6A | 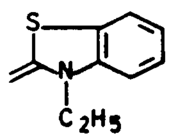 | 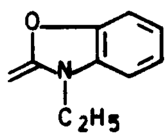 | 1,9 | 462 (Dimethylformamid) |
| 7A | 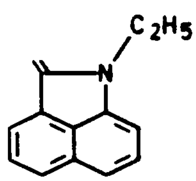 | $R^1$ | 2,6 | 670 ($CH_2Cl_2$) |
| 8A | 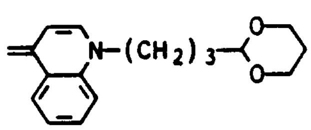 | $R^1$ | 6,1 | 670 ($CH_2Cl_2$) |
| 9A | 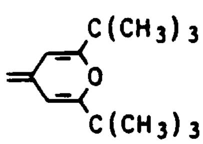 | $R^1$ | 9,8 | 600 (Toluol) |

b2) Herstellung von Photoleiterschichten

Die in Tabelle 7 aufgeführten und analog zu den Beispielen a) hergestellten Farbstoffe I wurden zur Herstellung von Photoleiterschichten eingesetzt.

Dazu wurde eine 10 gew.-%ige Lösung einer Mischung aus 1 Gew.-% des Farbstoffs I als Sensibilisator, 27 Gew.-% 2-(4'-Ethylphenylaminophenyl)-6-methoxybenztriazol-(1,2,3) und 18 Gew.-% 2,5-Bis-4,4-diethylamino-phenyloxdiazol-(1,2,3) als Ladungsträger transportierende Verbindungen und 55 Gew.-% eines

Copolymerisats aus 55 Gew.-% Styrol, 25 Gew.-% Acrylsäure, 15 Gew.-% Maleinsäureanhydrid und 5 Gew.-% Vinylacetat (K-Wert 36) als Bindemittel in einem Aceton/Tetrahydrofuran-Gemisch (1:1) 3 h bei Raumtemperatur gerührt.

Nach Filtration wurde die Lösung mit einer Rakel so auf ein feingebürstetes, 105 µm dickes Aluminiumblech aufgetragen, daß nach dem Ablüften des Lösungsmittels und dem Trocknen (15 min bei 80 °C) ein Auftrag mit einer Trockenschichtdicke von 4,0 ± 0,1 µm resultierte.

Die so beschichtete Platte wurde 25 h im Dunkeln gelagert und mittels einer Hochspannungscorona bis zum maximal möglichen Oberflächenpotential aufgeladen. Anschließend wurden der Potentialverlust in 20 sec im Dunkeln (Dunkelleitfähigkeit) sowie der durch Bestrahlung mit dem weißen Licht einer 100 W-XDO-Xenonhochdrucklampe aus einem Abstand von 45 cm in 10 sec induzierte Photopotentialabfall gemessen.

Tabelle 7

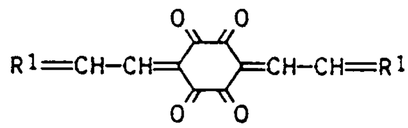

| Bsp. | R1 | Potentialverlust im Dunkeln [%] | Photopotentialabfall [%] |
|---|---|---|---|
| 10A | 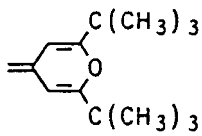 | 32,0 | 95,7 |
| 11A | 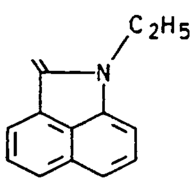 | 21,9 | 73,8 |
| 12A | 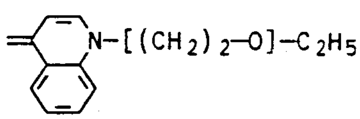 | 13,4 | 81,1 |

**Patentansprüche**

**1.** Polyketomethinfarbstoffe der allgemeinen Formel I

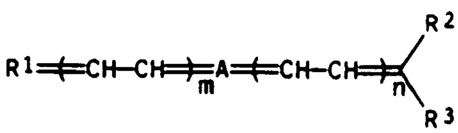 I

in der die Variablen folgende Bedeutung haben:

A eine Gruppierung der Formel A1 oder A2

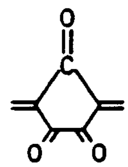 oder 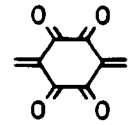

A1 A2

| | |
|---|---|
| $R^1$ | einen 5- oder 6-gliedrigen cycloaliphatischen Rest mit ein oder zwei Heteroatomen aus der Gruppe $-NR^4-$, -O-und -S-, der mit einer iso- oder heteroaromatischen Gruppe anelliert sein kann, wobei |
| $R^4$ | folgende Bedeutung hat: |
| | eine $C_5$-$C_7$-Cycloalkylgruppe oder eine Phenylgruppe, die Halogen, $C_1$-$C_4$-Alkyl oder -Alkoxy als Substituenten tragen kann; |
| | eine $C_1$-$C_{20}$-Alkylgruppe, deren C-Kette durch ein bis fünf Sauerstoffatome in Etherfunktion unterbrochen sein kann und die folgende Substituenten tragen kann: Hydroxy, Phenyl, 1,3-Dioxan-2-yl, 1,3-Dioxolan-2-yl, $-NHR^5$, $-NHCOR^5$, $-CONHR^5$, $-OCONHR^5$, $-N^{\oplus}(R^5)_3 \bullet An^{\ominus}$, $-SO_3H$ oder $-SO_3^{\ominus}Ka^{\oplus}$, |
| | wobei |
| $R^5$ | einen der unsubstituierten oder durch Hydroxy oder Phenyl substituierten Alkylreste $R^4$ oder Phenyl bedeutet und die Reste $R^5$ im Fall des Substituenten $-N^{\oplus}(R^5)_3$ gleich oder verschieden sein können, |
| $An^{\ominus}$ | für das Äquivalent eines Anions und |
| $Ka^{\oplus}$ | für das Äquivalent eines Kations stehen; |
| $R^2$, $R^3$ | gleiche oder verschiedene $C_1$-$C_{10}$-Alkylgruppen oder gemeinsam einen der Reste $R^1$, wobei im Fall der Gruppierung A1 Rebe $R^1$ der Formeln |

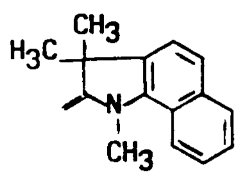 ; 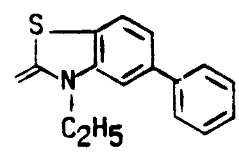 oder 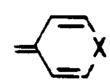

in denen X für Sauerstoff, Schwefel, Selen, $-N(CH_3)_2$ oder

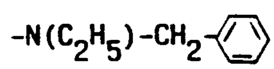

steht und die durch Alkyl-, Aryl- oder Aminogruppen substituiert sein können, ausgeschlossen sind, und
im Fall der Gruppierung A2 Reste $R^1$ der Formeln

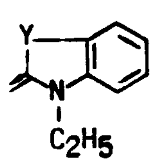 ; 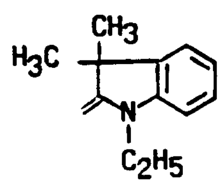 ; 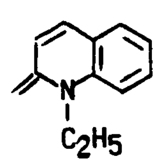 oder 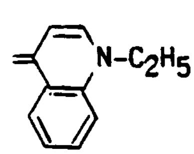

in denen Y für Sauerstoff oder Schwefel steht, ausgeschlossen sind;

| | |
|---|---|
| m, n | gleiche oder verschiedene ganzzahlige Werte von 0 bis 3. |

## Claims

**1.** Polyketomethine dyes of the general formula I

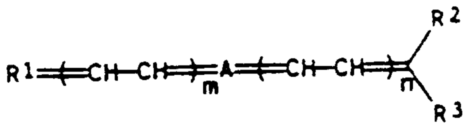

I

where

A is a group of the formula A1 or A2

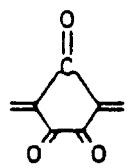

A1

or

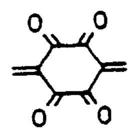

A2

$R^1$ is a 5- or 6-membered cycloaliphatic radical which contains one or two hetero atoms from the group $-NR^4-$, -O- and -S- and which may be fused to an isoaromatic or heteroaromatic group, where

$R^4$ is a $C_5$-$C_7$-cycloalkyl group or a phenyl group which may carry halogen, $C_1$-$C_4$-alkyl or -alkoxy as substituents, or
a $C_1$-$C_{20}$-alkyl group whose carbon chain may be interrupted by from one to five oxygen atoms in ether function and which may carry the following substituents: hydroxyl, phenyl, 1,3-dioxan-2-yl, 1,3-dioxolan-2-yl, $-NHR^5$, $-NHCOR^5$, $-CONHR^5$, $-OCONHR^5$, $-N^{\oplus}(R^5)_3 \cdot An^{\ominus}$, $-SO_3H$ or $-SO_3^{\ominus}Ka^{\oplus}$,
where

$R^5$ is one of the unsubstituted or hydroxyl-or phenyl-substituted alkyl radicals $R^4$ or phenyl and the radicals $R^5$ in the substituent $-N^{\oplus}(R^5)_3$ may be identical or different,

$An^{\ominus}$ is the equivalent of an anion, and

$Ka^{\oplus}$ is the equivalent of a cation,

$R^2$ and $R^3$ are identical or different $C_1$-$C_{10}$-alkyl groups or together one of the radicals $R^1$, provided that in the case of the group A1 radicals $R^1$ of the formulae

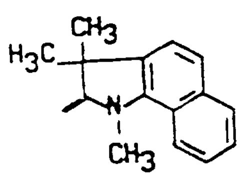

;

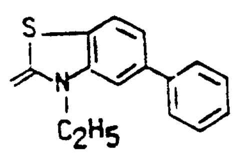

or

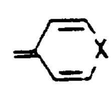

in which X is oxygen, sulfur, selenium, $-N(CH_3)_2$ or $-N(C_2H_5)$-$CH_2$ and which may be substituted by alkyl, aryl or amino groups shall be excluded and in the case of the group A2 radicals $R^1$ of the formulae

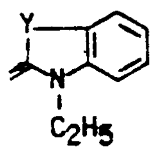 ; 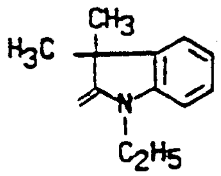 ; 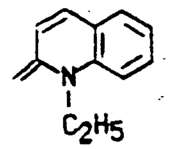 or 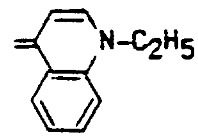

in which Y is oxygen or sulfur shall be excluded,

m and n are identical or different integers from 0 to 3.

## Revendications

**1.** Colorants au polycétométhine de formule générale

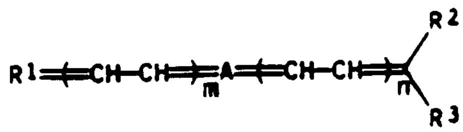 I

dans laquelle les variables ont les significations suivantes:

A Groupement de formule A1 ou A2

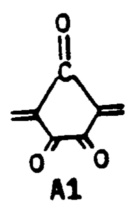
A1

ou

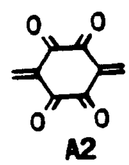
A2

$R^1$ Reste cycloaliphatique à 5 ou 6 termes qui renferme un ou deux hétéroatomes du groupe constitué par $-NR^4-$, -O- et -S- et qui peut être condensé avec un groupement iso- ou hétéroaromatique,

$R^4$ ayant la signification suivante:
Groupement cycloalkyle en $C_5$-$C_7$ ou groupement phényle qui peut porter des atomes d'halogène ou des restes alkyle ou alcoxy en $C_1$-$C_4$ en tant que substituants;
Groupement alkyle en $C_1$-$C_{20}$, dont la chaîne carbonée peut être interrompue par un à cinq atomes d'oxygène en fonction éther et qui peut porter les substituants suivants: hydroxy, phényle, 1,3-dioxanne-2-yle, 1,3-dioxolanne-2-yle, $-NHR^5$, $-NHCOR^5$, $-CONHR^5$, $-OCONHR^5$, $-N^{\oplus}(R^5)_3.An^{\ominus}$, $SO_3H$ ou $-SO_3^{\ominus}Ka^{\oplus}$, où

$R^5$ représente l'un des restes alkyle $R^4$ non substitués ou substitués par des groupements hydroxy ou phényle, ou un reste phényle, et les restes $R^5$ dans le cas du substituant $-N^{\oplus}(R^5)_3$ peuvent être identiques ou différents,

$An^{\ominus}$ est mis pour l'équivalent d'un anion et

$Ka^{\oplus}$ est mis pour l'équivalent d'un cation;

$R^2$, $R^3$ représentent des groupements alkyle en $C_1$-$C_{10}$ identiques ou différents ou forment ensemble l'un des restes $R^1$, à l'exclusion, dans le cas du groupement A1, des restes $R^1$ de formules

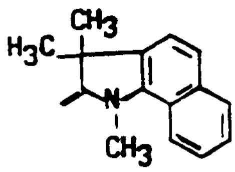 ; 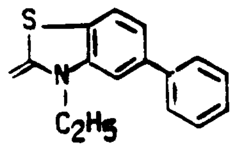 ou 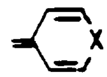

dans lesquelles X est mis pour un atome d'oxygène, de soufre, de sélénium ou pour un groupement $-N(CH_3)_2$ ou

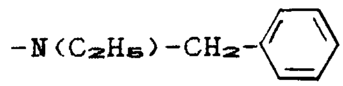

et qui peuvent être substitués par des groupements alkyle, aryle ou amino, et à l'exclusion, dans le cas du groupement $A^2$, des restes $R^1$ de formules

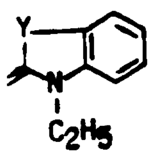 ; 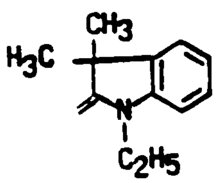 ; 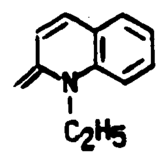 ou 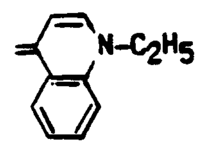

dans lesquelles Y est mis pour un atome d'oxygène ou de soufre;

m, n sont des nombres entiers identiques ou différents de 0 à 3.